(19) Europäisches Patentamt
European Patent Office
Office européen des brevets

(11) **EP 3 924 510 B1**

(12) **EUROPEAN PATENT SPECIFICATION**

(45) Date of publication and mention
of the grant of the patent:
**06.05.2026 Bulletin 2026/19**

(21) Application number: **20703067.7**

(22) Date of filing: **11.02.2020**

(51) International Patent Classification (IPC):
**C12Q 1/6809** (2018.01)

(52) Cooperative Patent Classification (CPC):
(C-Sets available)
**C12Q 1/6809** (Cont.)

(86) International application number:
**PCT/EP2020/053497**

(87) International publication number:
**WO 2020/165184 (20.08.2020 Gazette 2020/34)**

(54) **METHOD OF DETERMINING THE ORIGIN OF NUCLEIC ACIDS IN A MIXED SAMPLE**

VERFAHREN ZUR BESTIMMUNG DER HERKUNFT VON NUKLEINSÄUREN IN EINER
GEMISCHTEN PROBE

PROCÉDÉ DE DÉTERMINATION DE L'ORIGINE D'ACIDES NUCLÉIQUES DANS UN
ÉCHANTILLON MIXTE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **13.02.2019 EP 19156966**

(43) Date of publication of application:
**22.12.2021 Bulletin 2021/51**

(73) Proprietor: **MEDICOVER PUBLIC CO LTD
Nicosia 2409 (CY)**

(72) Inventors:
• **KOUMBARIS, George
2565 Lithrodontas (CY)**
• **ACHILLEOS, Achilleas
3313 Limassol (CY)**
• **IOANNIDES, Marios
2416 Nicosia (CY)**
• **PATSALIS, Philippos
2402 Nicosia (CY)**

(74) Representative: **CH Kilger Anwaltspartnerschaft
mbB
Fasanenstraße 29
10719 Berlin (DE)**

(56) References cited:
**WO-A1-2017/012592 WO-A1-2017/181146
WO-A1-2018/081130**

• **CHRISTOPHER K. RAYMOND ET AL: "Collection
of cell-free DNA for genomic analysis of solid
tumors in a clinical laboratory setting", PLOS
ONE, vol. 12, no. 4, 27 April 2017 (2017-04-27),
pages 1 - 11, XP055595111, DOI: 10.1371/
journal.pone.0176241**
• **HEITZER ELLEN ET AL: "Current and future
perspectives of liquid biopsies in genomics-
driven oncology", NATURE REVIEWS
GENETICS, NATURE PUBLISHING GROUP, GB,
vol. 20, no. 2, 8 November 2018 (2018-11-08),
pages 71 - 88, XP036675874, ISSN: 1471-0056,
[retrieved on 20181108], DOI: 10.1038/
S41576-018-0071-5**
• **MATTHEW?W. SNYDER ET AL: "Cell-free DNA
Comprises an In Vivo Nucleosome Footprint that
Informs Its Tissues-Of-Origin", CELL, vol. 164,
no. 1-2, 14 January 2016 (2016-01-14),
AMSTERDAM, NL, pages 57 - 68, XP055367434,
ISSN: 0092-8674, DOI: 10.1016/j.cell.2015.11.050**

• **MAXIM IVANOV ET AL: "Non-random fragmentation patterns in circulating cell-free DNA reflect epigenetic regulation", BMC GENOMICS, BIOMED CENTRAL LTD, LONDON, UK, vol. 16, no. 13, 16 December 2015 (2015-12-16), pages 1 - 12, XP021236698, DOI: 10.1186/1471-2164-16-S13-S1**

(52) Cooperative Patent Classification (CPC): (Cont.)

C-Sets
**C12Q 1/6809, C12Q 2525/191, C12Q 2537/159, C12Q 2565/519**

## Description

## FIELD OF THE INVENTION

The invention is in the field of biology, medicine and chemistry, in particular in the field of molecular biology and more in particular in the field of molecular diagnostics.

## BACKGROUND OF THE INVENTION

The discovery of cell-free fetal DNA (cffDNA) in maternal plasma has greatly promoted the development of non-invasive prenatal diagnosis. However, the concentration of cffDNA in maternal plasma varies among individuals, is extremely low and accounts for, in most cases, 2-19% of the total maternal plasma cell-free DNA (cfDNA). When the proportion of cffDNA in the maternal circulation is below 4%, even with next generation sequencing (NGS) technology, which has a high sensitivity, obtaining sufficient accuracy for non-invasive prenatal testing (NIPT) is challenging. Methods for detecting and monitoring diseases, such as cancer, based on the analysis of cfDNA fragmentation patterns are described in the international patent applications pub. No. WO 2018/081130 A1 and WO 2017/181146 A1.

## SUMMARY OF THE INVENTION

Eukaryotic genomes are organized into chromatin which enables not only to compact DNA but also regulates DNA metabolism (replication, transcription, repair, recombination). A current challenge is thus to understand (i) how functional chromatin domains are established in the nucleus, (ii) how chromatin structure/-information is dynamic through assembly, disassembly, modifications and remodeling mechanisms and (iii) how these events participate in and/or maintain disease establishment, progression and relapse. Understanding these events will allow identification of novel mechanisms of disease progression and new therapeutic targets, as well as controlling the effect of therapeutic molecules. It has been shown that signatures of chromatin structure in eukaryotic organisms, in particular the nucleosome arrangement, can be used to identify rare nucleic acid fragments in complex mixtures present in eukaryotic organisms (Heitzer E. et al. Nat. Rev. Genet. 2019 Feb;20(2):71-88). Such complex mixtures may be, for example, cffDNA and maternal cfDNA or, DNA derived from circulating tumor cells (CTCs) or tissue, and DNA derived from healthy circulating cells.

In particular, the inventors discovered a new method of isolating and identifying rare nucleic acids in mixed samples employing a novel targeted approach utilizing long synthetic TArget Capture Sequences (TACS) (probes) and novel bioinformatics and discovered that non-random fragmentation patterns exist in regions spanned by these TACS (probes).

If fragmentation is random then it would be equally likely to identify DNA fragments with start and/or stop positions at any base position spanned by the TACS (probes). This would lead to uniform-like coverage of fragments' start and/or stop positions across a probe location. Deviations from such coverage illustrate non-random fragmentation positions.

In order to identify such deviations, the following was performed:

1. A vector of genomic coordinates of all start and/or stop locations of all fragments that align within a probe-specific region (i.e. one probe) was created;
2. A density of start and/or stop location was created from the vector obtained in step 1. (i.e. a plot where the y-axis is frequency of occurrence and x-axis is coordinates spanning a single probe);
3. The deviation from uniform coverage from the density created from step 2 was assessed as this implies a non-random fragmentation mechanism.

A number of such positions per chromosome were discovered. The mechanism hypothesized to be responsible for the increased frequency of non-random fragmentation positions in certain regions is the protection of the DNA by the nucleosome. That is, deviation from uniform-like coverage may imply a reduced presence of a type of nucleic acid at such positions (e.g. an abundant nucleic acid comprising a complex mixture of nucleic acids) due to the protection conferred by the nucleosomal arrangement, and by extension an increased presence of other types of nucleic acid (e.g. a rare nucleic acid present in a complex mixture of nucleic acids), permitting the detection of regions with increased frequency of non-random fragmentation positions (referred to as hot spots for non-random fragmentation [HSNRF]).

HSNRF is hereby termed as a genomic region comprising, at a distance of less than 300 bp, (preferably less than 200 bp, more preferably less than 100 bp), preferred sites differentiating two tissue types present in a mixture of cfDNA, and where said preferred sites are present at a higher frequency in HSNRF regions than in other non-HSNRF regions. Preferred sites are hereby termed as genomic bases at which the frequency of being an end-point of a read is significantly different (p value of at least less than 0.05) between the two tissue types present in a mixture of cfDNA.

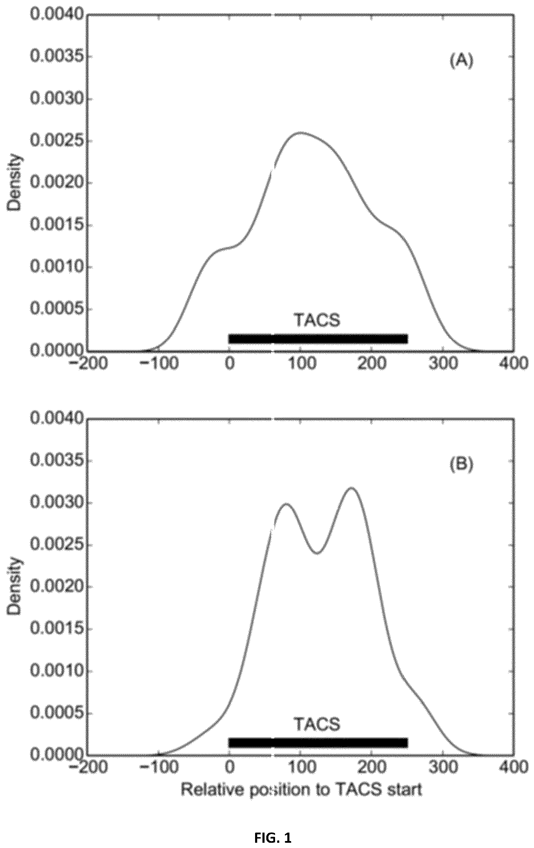

In one embodiment, deviation from uniform coverage was assessed by quantifying the number of modes in the distribution created from the start and/or stop positions. Figure 1 illustrates visually such an assessment, wherein a probe position is shown where no HSNRF has been detected (Figure 1A), whilst a probe position is shown where HSNRF has been detected (Figure 1B). Note the pronounced differences in the distribution of the start and/or stop position densities. The HSNRF exhibits a bimodal distribution. The ordinarily skilled artisan will appreciate that there are many ways of detecting such phenomena, including but not limited to, quantification of the maxima and minima of the distribution. In another embodiment, HSNRF can be detected with cluster analysis using the start/stop positions.

As such, in a first aspect the invention relates to a method for determining the origin of a nucleic acid fragment, or detecting a nucleic acid fragment, in a mixture of nucleic acid fragments, wherein the mixture comprises maternal and fetal cell-free DNA, or tumor and non-tumor cell-free DNA, the method comprising the steps of

(a) providing a mixture of fragmented nucleic acids stemming from a human subject,
(b) preparing a sequencing library from the mixture of fragmented nucleic acids,
(c) hybridizing one or more long probes to at least one location in said library, wherein the mixture of fragmented nucleic acids comprises a genomic region comprising, at a distance of less than 300 bp, genomic bases at which the frequency of being an end-point of a read is significantly different, with a p-value of less than 0.05, between two tissue types present in a mixture of cell-free DNA (cfDNA) and said probe covers said genomic region, wherein the probes are double-stranded probes and,

i. each probe is between 100-500 base pairs in length,
ii. each denatured probe has a 5'-end and a 3'-end,
iii. each probe binds to a genomic region comprising, at a distance of less than 300 bp, genomic bases differentiating two tissue types present in a mixture of cfDNA at least 10 base pairs away, on both the 5'-end and the 3'-end, from regions harboring copy number variations (CNVs), segmental duplications or repetitive DNA elements, and
iv. iv. the GC content of each probe is between 10% and 80%,

(d) isolating one or more fragmented nucleic acids from the mixture that are bound by the one or more probes,
(e) sequencing the enriched library wherein a duplication rate of the sequencing library from the template DNA fragments is more than 5%,
(f) determining the fragment size of a fragmented nucleic acid without alignment on any reference genome by using the sequence similarity of sequenced reads by detecting the amount of overlap and/or homology of paired-reads or the length of single reads
(g) determining the sequence of the, at least 20bp, outermost nucleotides of the fragmented nucleic acid and
(h) utilizing the information from steps (f) and (g) in order to

i. determine whether the size of the nucleic acid fragment is less than 150 bp, or
ii. determine whether the size of the nucleic acid fragment is greater than or equal to 150 bp and the sequence derived from step (g) overlaps said genomic region, and

(i) categorizing the fragment as of placental or tumor origin if the criteria in step (h)(i) or step (h)(ii) are met or categorizing the fragment as of maternal or non-tumor origin if neither of the criteria in step (h)(i) and step (h)(ii) are met.

The above method is designed to detect genomic regions with high probability of being a hotspot (many sites in a closed distance) of preferred/differential sites between two tissue types in a mixture of cfDNA fragments.

In a second aspect, the present invention provides a method for isolating one or more nucleic acid fragments from a mixture of nucleic acid fragments, comprising the steps of:

a. providing a mixture of fragmented nucleic acids, preferably DNAs, stemming from a human subject;
b. hybridizing one or more probes to at least one location in the nucleic acid fragments, where a genomic region comprising, at a distance of less than 300 bp, genomic bases at which the frequency of being an end-point of a read is significantly different, with a p-value of less than 0.05, between two tissue types present in a mixture of cfDNA lies, wherein:

i. each probe is between 100-500 base pairs in length;
ii. each denatured probe has a 5'-end and a 3'-end;
iii. each probe binds to a genomic region comprising, at a distance of less than 300 bp, genomic bases differentiating two tissue types present in a mixture of cfDNA at least 10 base pairs away, on both the 5'-end and the 3'-end, from regions harboring copy number variations (CNVs), segmental duplications or repetitive DNA elements; and
iv. the GC content of each probe is between 10% and 80%, or

c. amplifying one or more locations from the nucleic acid fragments, wherein the primers for the amplification lie adjacent to said genomic region.

In a third aspect, the present invention provides a kit for determining the origin of a nucleic acid fragment in a mixture of nucleic acid fragments for the use in a method according to the first aspect, comprising:

a. probes that hybridize to at least one location in the nucleic acid fragments, wherein said at least one location partially or completely encompasses a genomic region comprising, at a distance of less than 300 bp, genomic bases at which the frequency of being an end-point of a read is significantly different, with a p-value of less than 0.05, between two tissue types present in a mixture of cfDNA wherein:

i. each probe is between 100-500 base pairs in length;
ii. each denatured probe has a 5'-end and a 3'-end;
iii. each probe binds to a genomic region comprising, at a distance of less than 300 bp, genomic bases differentiating two tissue types present in a mixture of cfDNA at least 10 base pairs away, on both the 5'-end and the 3'-end, from regions harboring copy number variations (CNVs), segmental duplications or repetitive DNA elements; and
iv. the GC content of each probe is between 10% and 80%, and, optionally,

b. reagents and/or software for performing the determination and/or detection method.

## BRIEF DESCRIPTION OF THE FIGURES

Figure 1 shows an example of the distribution of nucleic acid fragment start and/or stop positions across probes, wherein in (A) the density is unimodal uniform-like across the probe coordinates, whilst in (B) the density is bimodal across the probe coordinates, thereby indicating the presence of a HSNRF. Note the differences in the distribution of the start and/or stop position densities.

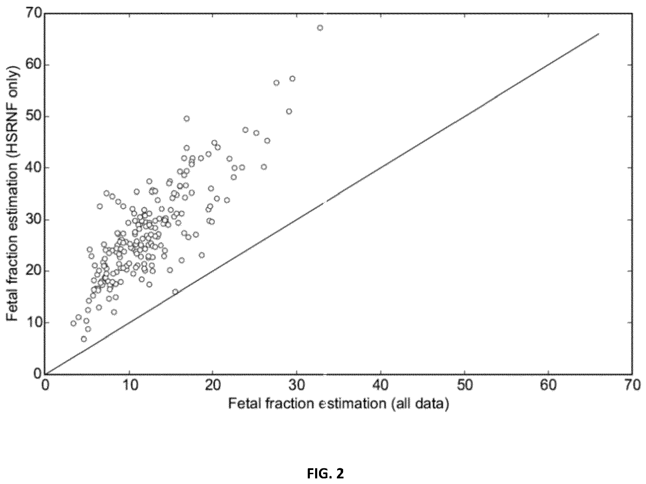

Figure 2 shows the enrichment of the minority fraction of a mixed sample when a sample is subjected to the HSNRF method on short fragments described herein and the minority fraction presence is subsequently assessed. Each dot in the figure represents a mixed sample, where the minority fraction is fetally-derived DNA and the majority fraction is maternally-derived DNA. The x-axis shows the minority fraction estimate before the HSNRF method on small fragments is applied and the y-axis shows the minority fraction estimate after the method is applied. The results illustrate an increase of the minority fraction indicating the presence of a greater amount of such DNA.

## DETAILED DESCRIPTION OF THE INVENTION

As such, in a first aspect the invention relates to a method for determining the origin of a nucleic acid fragment, or detecting a nucleic acid fragment, in a mixture of nucleic acid fragments, wherein the mixture comprises maternal and fetal cell-free DNA, or tumor and non-tumor cell-free DNA, the method comprising the steps of:

(a) providing a mixture of fragmented nucleic acids stemming from a human subject,
(b) preparing a sequencing library from the mixture of nucleic acid fragments
(c) hybridizing one or more probes to at least one location in said library, wherein the mixture of fragmented nucleic acids comprises a genomic region comprising, at a distance of less than 300 bp, genomic bases at which the frequency of being an end-point of a read is significantly different, with a p-value of less than 0.05, between two tissue types present in a mixture of cfDNA and said probe covers said genomic region, wherein the probes are double-stranded probes and,

i. each probe is between 100-500 base pairs in length,
ii. each denatured probe has a 5'-end and a 3'-end,
iii. each probe binds to a genomic region comprising, at a distance of less than 300 bp, genomic bases differentiating two tissue types present in a mixture of cfDNA at least 10 base pairs away, on both the 5'-end and the 3'-end, from regions harboring copy number variations (CNVs), segmental duplications or repetitive DNA elements, and
iv. the GC content of each probe is between 10% and 80%,

(d) isolating one or more fragmented nucleic acids from the mixture that are bound by the one or more probes,
(e) sequencing the enriched library wherein a duplication rate of the sequencing library from the template DNA fragments is more than 5%,
(f) determining the fragment size of the fragmented nucleic acids without alignment on any reference genome by using the sequence similarity of sequenced reads by detecting the amount of overlap and/or homology of paired-reads or the length of single reads,
(g) determining the sequence of the, at least 20bp, outermost nucleotides and
(h) utilizing the information from steps (f) and (g) in order to

i. determine whether the size of the nucleic acid fragment is less than 150 bp, or

ii. determine whether the size of the nucleic acid fragment is greater than or equal to 150 bp and the sequence derived from step (g) overlaps said genomic region, and

(j) categorizing the fragment as of placental or tumor origin if the criteria in step (h)(i) or step (h)(ii) are met or categorizing the fragment as of maternal or non-tumor origin if neither of the criteria in step (h)(i) and step (h)(ii) are met.

In the context of the present invention, step (c) defines hybridizing said sequencing library to one or more probes covering hot spots of non-random fragmentation (HSNRF) wherein said HSNFR regions are regions having, at a distance of less than 100-300 base pairs, preferred sites differentiating two tissue types present in a mixture of cfDNA, and where said preferred sites are present at a higher frequency in HSNRF regions than in other non-HSNRF regions. Preferred sites are hereby termed as genomic bases at which the frequency of being an end-point of a read is significantly different between the two tissue types present in a mixture of cfDNA.

The above method is designed to detect genomic regions with high probability of being a hotspot (many sites in a closed distance) of preferred and/or differential sites between two tissue types in a mixture of cfDNA fragments.

HSNRF is hereby termed as a genomic region comprising, at a distance of less than 300 bp, (preferably less than 200 bp, more preferably less than 100 bp), preferred sites differentiating two tissue types present in a mixture of cfDNA, and where said preferred sites are present at a higher frequency in HSNRF regions than in other non-HSNRF regions. Preferred sites are hereby termed as genomic bases at which the frequency of being an end-point of a read is significantly different (p value of at least less than 0.05) between the two tissue types present in a mixture of cfDNA.

Herein, the mixture of nucleic acid fragments is preferably isolated from a sample taken from a eukaryotic organism, preferably a primate, more preferably a human.

In the context of the present invention, the term probe refers to synthetic TArget Capture Sequences (TACS).

In the context of the present invention, the expression "nucleic acid fragments" and "fragmented nucleic acids" can be used interchangeably.

In a preferred embodiment of the method according to the invention, the nucleic acid fragments are circulating cell-free DNA or RNA.

In one embodiment, the DNA sample is a maternal plasma sample comprising maternal DNA and cell-free fetal DNA (cffDNA).

In another embodiment, the DNA sample (e.g. mixture of nucleic acid fragments) comprises cell-free tumor DNA (cftDNA). In one embodiment, the DNA sample is selected from the group consisting of a plasma sample, a urine sample, a sputum sample, a cerebrospinal fluid sample, an ascites sample and a pleural fluid sample from a subject having or suspected of having a tumor. In one embodiment, the DNA sample is from a tissue sample from a subject having or suspected of having a tumor.

The methods of the invention can be used with a variety of biological samples. Essentially any biological sample containing genetic material, e.g. RNA or DNA, and in particular cell-free DNA (cfDNA), can be used as a sample in the methods allowing for genetic analysis of the RNA or DNA therein. For example, in one embodiment, the DNA sample is a plasma sample containing cell-free DNA (cfDNA). In particular, for prenatal testing the DNA sample contains fetal DNA (e.g., cell-free fetal DNA). In one embodiment for NIPT, the sample is a mixed sample that contains both maternal DNA and fetal DNA (e.g., cell-free fetal DNA (cffDNA)), such as a maternal plasma sample obtained from maternal peripheral blood. Typically for mixed maternal/fetal DNA samples, the sample is a maternal plasma sample, although other tissue sources that contain both maternal and fetal DNA can be used. As used herein, the term "mixed sample" refers to a mixture of at least two biological samples originating from different sources, i.e. maternal/fetal DNA samples.

Depending upon the circumstances, the biological sample encompasses: embryonic DNA and maternal DNA, tumor derived DNA and non-tumor derived DNA, pathogen DNA and host DNA and DNA derived from a transplanted organ and DNA derived from the host.

Therefore, in the context of non-invasive diagnosis, the sample is a mixed sample, wherein said mixed sample is selected from the group comprising (i) embryonic DNA and maternal DNA, (ii) tumor derived DNA and non-tumor derived DNA, (iii) pathogen DNA and host DNA and (iv) DNA derived from a transplanted organ and DNA derived from the host.

In one embodiment the fetal genetic material contribution is smaller than the maternal contribution. In one embodiment the average length of fragments originating from the placenta is < 140-150 base pairs and the average length of maternally derived fragments is ≥ 160 base pairs. The fetal material contribution can be assessed using information of genetic loci differences that exist between the fetal and maternal genomes, such as but not limited to single nucleotide polymorphisms (SNPs).

In one embodiment, minor allele frequency (MAF) values (0-50%) are used to assess the fetal contribution. In another embodiment, all detected SNPs are utilized to estimate the contribution of the fetal component.

Maternal plasma can be obtained from a peripheral whole blood sample from a pregnant subject and the plasma can be obtained by standard methods. As little as 1-4 ml of plasma is sufficient to provide suitable DNA material for analysis according to the method of the disclosure. Total cell-free DNA can then be extracted from the sample using standard techniques, non-limiting examples of which include a QIAsymphony protocol (QIAGEN) suitable for cell-free fetal DNA isolation or any other manual or automated extraction method suitable for cell-free DNA isolation.

In yet another embodiment for oncology purposes, the sample is a biological sample obtained from a subject having or suspected of having a tumor. In one embodiment, the DNA sample comprises cell-free tumor DNA (cftDNA). In another embodiment the sample is a subject's urine, sputum, ascites, cerebrospinal fluid or pleural effusion. In another embodiment, the oncological sample is a subject plasma sample, prepared from subject peripheral blood. Thus, the sample can be a liquid biopsy sample that is obtained non-invasively from a subject's blood sample, thereby potentially allowing for early detection of cancer prior to development of a detectable or palpable tumor, or allowing monitoring of disease progression, disease treatment, or disease relapse.

In the context of the present invention, the term "subject" refers to animals, preferably mammals, and, more preferably, humans. The "subject" referred to herein is a pregnant subject, and, therefore, preferably, a female subject. The pregnant subject may be at any stage of gestation. The "subject" may get pregnant naturally or by means of artificial techniques. As used herein, the term "subject" also refers to a subject suffering from or suspected of having a tumor. Said subject can be subjected to organ transplantation or experienced a pathogen infection after a transplant or independently from a transplant.

For the biological sample preparation, typically, DNA is extracted using standard techniques known in the art, a non-limiting example of which is the QIAsymphony (QIAGEN) protocol.

Following isolation, the cell-free DNA of the sample is used for sequencing library construction to make the sample compatible with a downstream sequencing technology, such as Next Generation Sequencing. Typically, this involves ligation of adapters onto the ends of the cell-free DNA fragments. Sequencing library preparation kits are commercially available or can be developed.

Preferably, in the method according to the invention, the first and second nucleic acid fragments are selected from the groups comprising:

    i. embryonic DNA and maternal DNA,
    ii. tumor derived DNA and non-tumor derived DNA,
    iii. pathogen DNA and host DNA,
    iv. DNA derived from a transplanted organ and DNA derived from the host.

In the context of the present invention, the terms fetus and embryo are used interchangeably.

An HSNRF is usually associated with two fragment ends, one associated with the 5' end of a fragmented nucleic acid and one with the 3' end of a fragmented nucleic acid.

In one embodiment, preferably the probe spans a HSNRF site such that only the 5' end of the fragmented nucleic acid is captured by the probe.

In another embodiment, the probe spans HSNRF site such that only the 3' end of the cell-free nucleic acids arising from HSNRF can bind to the probe.

In another preferred embodiment, the probe spans both HSNRF sites associated with a fragmented nucleic acid such that both the 5' and the 3' end of a cell-free nucleic acid associated with the given HSNRF site are captured by the probe.

In another embodiment, mixtures of the above are used.

Ideally, in the method according to the invention, the TArget Capture Sequences (TACS) (probes) in step (c) of the description of the method found in the beginning of this section are long probes and, (i) each of the TACS (probes) is between 100-500 base pairs in length, (ii) each probe has a 5' end and a 3' end, (iii) preferably each probe binds to the HSNRF at least 10 base pairs away, on both the 5' end and the 3' end, from regions harboring copy number variations (CNVs), segmental duplications or repetitive DNA elements, and (iv) the GC content of each probe is between 19% and 80%.

In general, the probe-hybridization step, can be carried out before the sequencing library is created or after the library has been created.

The region(s) of interest on the chromosome(s) of interest where the HSNRF lie are enriched by hybridizing the pool of HSNRF-capture probes to the sequencing library, followed by isolation of those sequences within the sequencing library that bind to the probes. In one embodiment, the probe spans a HSNRF site such that only the 5' end of the fragmented cell-free nucleic acids is captured by the probe. In another embodiment the probe spans a HSNRF site such that only the 3' end of the fragmented cell-free nucleic acids arising from HSNRF can bind to the probe. In another preferred embodiment, the probe spans both HSNRF sites associated with a fragmented nucleic acid such that both the 5' and the 3' end of a cell-free nucleic acid associated with the given HSNRF site are captured by the probe.

To facilitate isolation of the desired, enriched sequences (HSNRF), typically the probe sequences are modified in such a way that sequences that hybridize to the probes can be separated from sequences that do not hybridize to the probes. Typically, this is achieved by fixing the probes to a support. This allows for physical separation of those sequences that bind the probes from those sequences that do not bind the probes. For example, each sequence within the pool of probes can be labeled with biotin and the pool can then be bound to beads coated with a biotin-binding substance, such as streptavidin or avidin. In a preferred embodiment, the probes are labeled with biotin and bound to streptavidin-coated magnetic beads, thereby allowing separation by exploiting the magnetic property of the beads. The ordinarily skilled artisan will appreciate, however, that other affinity binding systems are known in the art and can be used instead of biotin-streptavidin/avidin. For example, an antibody-based system can be used in which the probes are labeled with an antigen and then bound to antibody-coated beads. Moreover, the probes can incorporate on one end a sequence tag and can be bound to a support via a complementary sequence on the support that hybridizes to the sequence tag. Furthermore, in addition to magnetic beads, other types of supports can be used, such as polymer beads and the like.

In certain embodiments, the members of the sequencing library that bind to the pool of probes are fully complementary to the probe. In other embodiments, the members of the sequencing library that bind to the pool of probes are partially complementary to the probe. For example, in certain circumstances it may be desirable to utilize and analyze data that are from DNA fragments that are products of the enrichment process but do not necessarily belong to the genomic regions of interest (i.e. such DNA fragments could bind to the probe because of partial homologies) and when sequenced would produce very low coverage throughout the genome across non-probe coordinates.

Following enrichment of the sequence(s) of interest using the probes, thereby forming an enriched library of DNAs with HSNRF sites, the members of the enriched HSNRF library are eluted and are amplified and sequenced using standard methods known in the art. Reference (buffy coat) sonicated samples are used as normalisers, in addition to GC content, mappability and other technical artifacts adjustments, to clean the data and filter out false positive results.

Next Generation Sequencing (NGS) is typically used, although other sequencing technologies can also be employed, which provide very accurate counting in addition to sequence information.

Accordingly, other accurate counting methods, such as digital PCR, single molecule sequencing, nanopore sequencing, and microarrays can also be used instead of NGS.

As shown in more detail the HSNRF, i.e. its exact fragment size and cutting site, serves to validate the origin of the nucleic acid.

The invention relates to a method according to any of the aspects or embodiments, wherein the nucleic acid fragments to be detected or the origin of which is to be determined, is present in the mixture at a concentration lower than a nucleic acid fragment from the same genetic locus but of different origin. That means that if a particular locus is selected, e.g. the maternal copy will be present 100 times and the copy from the fetus only once, in the solution comprising the isolated cfDNA. In the case of fetal derived cfDNA, the fetal derived component of a mixed sample can have a range of possible values. For example, the range of fetal material in a mixed sample can be in the range of 2% - 30%. Frequently, fetal derived fragments are around 10% of the total DNA of a mixed sample. More importantly, in some compositions of mixed samples the fetal DNA component of the sample can be less than 5%. Particularly, in some sample compositions the fetally-derived material is 3%, or less, of the total sample.

The present method is particularly suited to analyze such low concentrations of target cfDNA. In the method according to the invention, the nucleic acid fragment to be detected or the origin of which is to be determined and the nucleic acid fragment from the same genetic locus but of different origin are present in the mixture at a ratio selected from the group of 1:2, 1:4, 1:10, 1:20, 1:50, 1:100, 1:200, 1:500, 1:1000, 1:2000 and 1:5000. The ratios are to be understood as approximate ratios which means plus/minus 30%, 20% or 10%. A person skilled in the art knows that such ratios will not occur at exactly the numerical values cited above. The ratios refer to the number of locus-specific molecules for the rare type to the number of locus-specific molecules for the abundant type.

In one embodiment, the probes are provided in a form that allows them to be bound to a support, such as biotinylated probes. In another embodiment, the probes are provided together with a support, such as biotinylated probes provided together with streptavidin-coated magnetic beads.

In a particular embodiment, the GC content of the probes is between 10% and 80%, preferably 15% and 60%, more preferably 20% and 50%.

In a second aspect, the present invention provides with a method for isolating one or more nucleic acid fragments from a mixture of nucleic acid fragments, comprising the steps of:

a. providing a mixture of fragmented nucleic acids, preferably DNA, stemming from a human subject;
b. hybridizing one or more probes to at least one location in the nucleic acid fragments, where a genomic region comprising, at a distance of less than 300 bp, genomic bases at which the frequency of being an end-point of a read is significantly different, with a p-value of less than 0.05, between two tissue types present in a mixture of cfDNA lies, wherein

i. each probe is between 100-500 base pairs in length;
ii. each denatured probe has a 5'-end and a 3'-end;
iii. each probe binds to a genomic region comprising, at a distance of less than 300 bp, genomic bases differentiating two tissue types present in a mixture of cfDNA at least 10 base pairs away, on both the 5'-end and the 3'-end, from regions harboring copy number variations (CNVs), segmental duplications or repetitive DNA elements; and

iv. the GC content of each probe is between 10% and 80%,

or

c. amplifying one or more locations from the nucleic acid fragments, wherein the primers for the amplification lie adjacent to said genomic region.

In a third aspect, the invention provides kits for carrying out the methods of the disclosure comprising:

a. probes that hybridize to at least one location in the nucleic acid fragment, wherein said at least one location partially or completely encompasses a genomic region comprising, at a distance of less than 300 bp, genomic bases at which the frequency of being an end-point of a read is significantly different, with a p-value of less than 0.05, between two tissue types present in a mixture of cfDNAwherein:

i. each probe is between 100-500 base pairs in length;
ii. each denatured probe has a 5'-end and a 3'-end;
iii. each probe binds to a genomic region comprising, at a distance of less than 300 bp, genomic bases differentiating two tissue types present in a mixture of cfDNA at least 10 base pairs away, on both the 5'-end and the 3'-end, from regions harboring copy number variations (CNVs), segmental duplications or repetitive DNA elements; and
iv. the GC content of each probe is between 10% and 80%, two tissue types present in a mixture of cfDNA and, optionally,

b. reagents and/or software for performing the determination and/or detection method.

In the context of the present invention, the term "partially" refers to a region (location) of 10, 20, 30 or 40 bases of the nucleic acid fragment from the 5'-end or from the 3'-end. Consequently, as used herein, the term "completely" refers to a region (location) which encompasses 100% of the nucleic acid fragment. According to the method of the present invention, the probes hybridize to at least one location within the nucleic acid fragment. However, more than one location within the same nucleic acid fragment can be also targeted by the probes.

In one embodiment, the kit comprises a container consisting of the pool of probes and software and instructions for performing the method.

In addition to the pool of probes, the kit can comprise one or more of the following (i) one or more components for isolating cell-free DNA from a biological sample, (ii) one or more components for preparing and enriching the sequencing library (e.g., primers, adapters, buffers, linkers, DNA modifying enzymes, ligation enzymes, polymerase enzymes, probes and the like), (iii) one or more components for amplifying and/or sequencing the enriched library, and/or (iv) software for performing statistical analysis.

In tumor samples determining the origin of a first nucleic acid fragment can be very important. The inventors have found that different tissues have different "signatures". Thus, it is possible to detect the origin of a first nucleic acid from a specific tissue, e.g. a tumor specimen.

For detection of tumor biomarkers probes are designed based on the design criteria described herein and the known sequences of tumor biomarker genes and genetic mutations therein associated with cancer. In one embodiment, a plurality of probes used in the method bind to a plurality of tumor biomarker sequences of interest. Here, the probe may lie in the hot spots of non-random fragmentation adjacent to the mutation site.

The biomarkers in such assay may be selected from the group comprising ABL, AKT, AKT1, ALK, APC, AR, ARAF, ATM, BAP1, BARD1, BCL, BMPR1A, BRAF, BRCA, BRCA1, BRCA2, BRIP1, CDH1, CDKN, CHEK2, CTNNB1, DDB2, DDR2, DICER1, EGFR, EPCAM, ErbB, ErcC, ESR1, FANCA, FANCB, FANCC, FANCD2, FANCE, FANCF, FANCG, FANCI, FANCL, FANCM, FBXW7, FGFR, FLT, FLT3, FOXA1, FOXL2, GATA3, GNA11, GNAQ, GNAS, GREM1, HOX, HOXB13, HRAS, IDH1, JAK, JAK2, KEAP1, KIT, KRAS, MAP2Ks, MAP3Ks, MET, MLH1, MPL, MRE11A, MSH2, MSH6, MTOR, MUTYH, NBN, NPM1, NRAS, NTRK1, PALB2, PDGFRs, PI3KCs, PMS2, POLD1, POLE, POLH, PTEN, RAD50, RAD51C, RAD51D, RAF1, RB1, RET, RUNX1, SLX4, SMAD, SMAD4, SMARCA4, SPOP, STAT, STK11, TP53, VHL, XPA,XPC, and combinations thereof.

In one embodiment of the method, following the library preparation and enrichment for the sequences of interest through probe hybridization, the subsequent step of amplifying the enriched library is performed in the presence of blocking sequences that inhibit amplification of wild-type sequences. Thus, amplification is biased toward amplification of the mutant tumor biomarker sequences.

In another embodiment, unique molecular barcodes may be used to label each DNA or RNA fragment

The pool of probes used in the method of detecting tumor biomarkers can include any of the design features described herein with respect to the design of the probes.

Suitable statistical analysis approaches for use with prenatal samples and oncology samples that enable detection of HSNRF biomarkers are described further in the Examples section.

Specific types of cancer can be characterized by and/or associated with DNA fragments in plasma having smaller size than the expected size of DNA fragments originating from healthy tissues. The same hypothesis holds true for fragments originating from the placenta/fetus. Specifically, placenta derived fragments are generally of smaller size compared to fragments originating from maternal tissues/cells. The present invention makes such an analysis possible in a novel manner. The probes enable the isolation and enrichment of low frequency, shorter fragments. Thus, the present method allows for a fragments-based detection of abnormalities in mixed samples with low signal-to-noise ratio.

Accordingly, in another embodiment of the invention, it is preferred that the fragment to be detected or determined is of smaller or larger size than the second fragment. Depending on the tissue of origin, different fragment sizes are expected across HSNRF locations. Since cells of each tissue are differentiated to perform specific functions, then different patterns of genetic activity are expected to be seen by each tissue type. Since gene activation is dependent on the tertiary structure of DNA then different tissues will have slightly different chromatin conformations which lead to different cut sites and consequently different fragment sizes. For example, it has been demonstrated that cell-free DNA of placental origin is likely to be of smaller size when compared to cell-free DNA of maternal origin.

The method described herein relies on the hypothesis that preferred sites exist but can differ between biological samples. By designing a method to perform online, per sample, estimation of HSNRF instead of using an offline reference set of preferred sites, the signal to noise ratio per sample is further improved (Figure 2).

**EXAMPLES**

The present invention is further illustrated by the following examples, which should not be construed as further limiting.

*Example 1: Sample collection and library preparation*

The general methodology for this probe-based approach of discovering HSNRF for non-invasive prenatal diagnosis purposes is explained. In this example, methods for collecting and processing a maternal plasma sample (containing maternal and fetal DNA) are described. The same approach can be followed for the discovery of HSNRF in other medically useful cases, such as, but not limited to oncology, genetic mutation, transplantation, and assessment of pathogen load.

*Sample collection*

Plasma samples were obtained anonymously from pregnant women after the 10th week of gestation. Protocols used for collecting samples were approved by the National Bioethics Committee, and informed consent was obtained from all participants.

*Sample extraction*

Cell-free DNA was extracted fromplasma from each individual using a manual or automated extraction method suitable for cell-free DNA isolation such as for example, but not limited to, QIAsymphony protocol suitable for cf DNA isolation (QIAGEN).

*Sequencing library preparation*

Extracted cell-free DNA from maternal plasma samples was used for sequencing library construction. A negative control extraction library was prepared separately to monitor any contamination introduced during the experiment. Initially, 5' and 3' overhangs were repaired while 5' ends were phosphorylated. Reaction products were purified using AMPure XP beads (Beckman Coulter). Subsequently, sequencing adaptors were ligated to both ends of the DNA, followed by purification using AMPure XP beads (Beckman Coulter). Nicks were removed in a fill-in reaction with a polymerase and were subsequently purified using AMPure XP beads (Beckman Coulter). Library amplification was performed using another polymerase enzyme (Koumbaris et al. (2016) Clinical chemistry 62(6):848-855). The final library products were purified using AMPure XP beads (Beckman Coulter) and measured by spectrophotometry.

*Example 2: TArget Capture Sequences (TACS) Design and Preparation*

This example describes preparation of custom TACS (probes) for the detection of HSNRF. The genomic target-loci used for TACS design were selected based on their GC content and their distance from repetitive elements (minimum 50 bp away). TACS size can be variable. In one embodiment of the method the TACS range from 100-500 bp in size and are generated as described below. The TACS were prepared by polymerase chain reaction (PCR) using Taq polymerase, primers designed to amplify the target-loci, and normal DNA as template. In a preferred embodiment, the TACS span an HSNRF site such that only the 5' end of the fragmented nucleic acid is captured by the probe. In another embodiment, TACS span an HSNRF site such that only the 3' end of the cell-free nucleic acids arising from HSNRF can bind to the probe. In another preferred embodiment, TACS span both HSNRF sites associated with a fragmented nucleic acid such that both the 5' and the 3' end of a cell-free nucleic acid associated with the given HSNRF site are captured by the TACS. PCR products were verified via agarose gel electrophoresis and purified using standard PCR clean up kits. Concentration was measured by spectrophotometry.

*Example 3: TACS hybridization and amplification*

This example describes the method of target capture of nucleic acids by hybridization using TACS, followed by sequencing of captured sequences by Next Generation Sequencing (NGS).

*TACS biotinylation*

TACS were prepared for hybridization, starting with blunt ending followed by purification. They were then ligated with a biotin adaptor and purified. TACS were denatured prior to immobilization on streptavidin coated magnetic beads.

*TACS hybridization*

Amplified libraries were mixed with blocking oligos, Cot-1 DNA, Salmon Sperm DNA, hybridization buffer, blocking agent, and were then denatured. Denaturation was followed by 30 minutes incubation at 37°C. The resulting mixture was then added to the biotinylated TACS and incubated for 12-48 hours at 60-70°C. After incubation, the enriched samples were washed as described previously and DNA was eluted by heating. Eluted products were amplified using outer-bound adaptor primers. Enriched amplified products were pooled equimolarly and sequenced on a suitable platform.

If appropriate, amplification may be intentionally biased toward amplification of specific/desired sequences. In one embodiment of the method, this is performed when amplification is performed in the presence of sequences that hybridize to the undesired sequence of interest, and as such block the action of the polymerase enzyme during the process. Hence, the action of the amplification enzyme is directed toward the sequence of interest during the process.

*Example 4: Detection of HSNRF candidates based on fragment start/stop positions*

This example illustrates the detection of HSNRF from sequencing data of maternal plasma samples, whereby the plasma sample is a mixed sample of maternally derived and fetally derived DNA.

Next generation sequencing (NGS) data was processed using standard methods to those versed in the art. Briefly, the NGS data were subject to a demultiplexing procedure whereby sequenced DNA fragments were assigned to their samples, as identified via an index sequence, producing FASTQ files.

In a preferred embodiment, each sample's FASTQ files were not aligned against a version of the human reference genome. The fragment-size information an/or class was obtained using a method of detecting the amount of overlap/homology of paired-reads or the length of single reads (when greater than 150bp). Identical reads are removed and either de-novo assembly or matching with pre-determined targets of short sequences was performed. The ordinarily skilled artisan will appreciate that whole genome alignment-based techniques are not highly accurate in low sequence identity regions and assume a genome wide linear order of homology which is not always true in case of sequence rearrangements.

The designed method performs an online, per sample, estimation of HSNRF instead of using an offline reference set of preferred sites, resulting in a very high signal to noise ratio per sample (Figure 2).

In another embodiment, each sample's FASTQ files were aligned against a version of the human reference genome using the Burrows-Wheeler alignment algorithm producing a text-based format file containing the information; the SAM file. Prior to further processing the SAM file was converted to a binary format to produce the BAM file. If appropriate, data originating from the same sample but found across different files where merged. Where necessary, data was curated for quality of sequencing and presence of duplicate DNA fragments to create a final BAM file. Data related to read-depth, fragment size and sequence information was retrieved from the final BAM file.

The ordinarily skilled artisan will appreciate that there exist many freely available and well-established tools to perform the aforementioned procedures.

*Hot spot for non-random fragmentation (HSNRF) detection*

The assumption for the discovery of HSNRF is that if fragmentation is random, and in the absence of sequencing biases, then it is equally likely to find DNA fragments with start and/or stop positions in all coordinates comprising the region spanned by a probe. More specifically, the distribution of start/stop coordinates within the probe is constructed and any deviations from a unimodal distribution are hypothesized to be indicative of non-random fragmentation, after adjusting for sequencing and GC-content biases using a set of randomly-fragmented samples.

To estimate the distribution of start/stop positions of fragments within a probe, we used nonparamentric kernel density estimation with an Epanechnikov kernel and a bandwidth estimated using, but not limited to, the Sheather and Jones approach. Subsequently, the obtained information was assessed to determine if there exists deviation from a unimodal distribution. Since the obtained information is describing the distribution of start and/or stop positions of DNA fragments within a probe, distribution relevant metrics can be used to assess deviation from uniform coverage. A non-limiting example of such an assessment is the identification of modes of a distribution. A multi-modal distribution is an example of a non-uniform distribution that is relevant to the present invention.

Figure 1 illustrates visually such an assessment. In panel (A), a probe position is shown where no HSNRF has been detected, whilst in panel (B) a probe position is shown where non-random fragmentation has been detected. Note the differences in the distribution of the start and/or stop position densities. In the presented example, the probe associated with non-random fragmentation illustrates a bimodal distribution implying the presence of protected regions; regions where there was reduced fragmentation and thus non-random fragmentation. This makes the probe a suitable candidate for existence of HSNRF.

A person trained in the art will appreciate that the assessment of modes of a distribution is an exemplary method of assessment of deviation from uniform and/or expected coverage. As such, the example of a multi-modal distribution is not to be construed as limiting the scope of the assessment. Other ways of detecting such phenomena exist, including but not limited to, quantification of the maxima and minima of the distribution.

*Example 5: Detection of HSNRF based on fragment size*

One property of HSNRF is the size of each nucleic acid fragment arising from such hot spots. The size may be a property that is associated with tissue of origin such as for example a cancerous tissue or a fetal related organ such as the placenta. Nucleic acid fragments arising from a cancerous tissue and nucleic acid fragments arising from a fetal tissue such as the placenta are in general of smaller size when compared to other nucleic acid fragments found in circulation in the body of a human subject.

Given that DNA fragmentation patterns are associated with a particular tissue of origin, HSNRF sites must be also associated with a particular fragment size and fragment size distribution. In the case of fetal material originating from the placenta, a hot spot can be described as a genomic coordinate where fragment ends cluster and the fragment size associated with the given cluster position is generally of smaller size, when compared to other nucleic acid fragments found in circulation of the plasma of a pregnant woman. To assess this, each base position part of the probe can be queried if a fragment starts and/or stops from the specific position and if such fragment exists, proceed with determining the size of the fragment. The size of a fragment can be obtained either before or after the alignment step.

This invention allows a different definition of small/large fragment per sample by allowing the threshold imposed to fragment size to vary with samples (depending on fetal fraction and distribution of fragment sizes). Depending on the tissue of origin, different fragment sizes are expected across HSNRF locations. Since cells of each tissue are differentiated to perform specific functions, then different patterns of genetic activity are expected to be seen by each tissue type. Since, gene activation is dependent on the tertiary structure of DNA then different tissues will have slightly different conformations which lead to different cut sites and consequently different fragment sizes.

In one embodiment, the size of all fragments that start and/or stop at the particular coordinate is then assessed to obtain a statistical measure that defines the expected fragment size. In one embodiment, fragment size is assessed using statistical properties of the fragment size distribution such as the mean, median or other quantile or mode of the distribution. A person skilled in the art can appreciate that other methods may be used to assess the distribution. If the statistical measure is below or above a given lower or upper bound then the given base position can be considered as a candidate hot spot associated with nucleic acids of placental (fetal) origin.

In one embodiment of the method, the enriched sample is sequenced using a 2x75 bp sequencing method, to allow collection of the start/end positions of the sequenced fragment both from the 5' and 3' end, as well as to obtain enough sequencing information of the fragment, to allow de novo/self-alignment. In said embodiment, sequenced fragments are grouped according to size, based on the extend of overlap or absence of overlap of their paired end reads. Each fragment is classified accordingly in two groups, short (group 1) and long (group 2). The HSNRF are identified by utilizing the sequence similarity of the at least 20 outermost nucleotides of fragments in group 1. Fragments from group 2, whose ends overlap with identified HSNRF are then used to construct group 3. Then all fragments from group 1, and all fragments from group 3 are retained for subsequent analysis. Said cfDNA fragments of group 1 are shorter in size and are enriched for cffDNA. Said cfDNA fragments of group 3 are longer in size but are also enriched for cffDNA because their ends overlap with HSNRF. Cell-freeDNA fragments of group 2 whose ends do not overlap with HSNRF have higher likelihood of being derived from the mother and are discarded from all subsequent analysis.

In another embodiment, a size threshold is predefined as 150 base pairs. Then each base position part of a probe is assessed to ascertain if a fragment starts and/or stops from such position and if so, the size of the fragment is compared against the threshold to determine if the fragment is small or large. The recordation is repeated for all positions of the probe in order to obtain an association between probe position and number of small-fragments originating from such position. Positions associated with a greater number of such small-fragments, when compared to other positions within the probe, are candidate hot spot positions associated with nucleic acids of placental (fetal) origin. The ordinarily trained artisan will appreciate that non-limiting examples of statistical measures and cluster analysis methods that can be used to identify candidate hot spots include the first and second moments of a distribution.

Probes may harbor many such hot spots of short or long fragments. In a mixed maternal sample, a method that uses kernel density estimation can be used to identify HSNRF that consist of fragments primarily originating from the mother and HSNRF of fragments primarily originating from the fetus. The hypothesis to be tested is that if a probe harbors maternally derived HSNRF, i.e. Regions in which the natural fragmentation pattern is alterned due to nucleosome occupancies, we should be able to detect more easily HSNRF associated with DNA fragments of fetal origin (short) in regions where the cutting sites of maternally derived fragments are of lower abundance. If this is true then the probes with maternally derived HSNRF should statistically have a greater number of HSNRF associated with short fragments, when compared to a probe that does not harbor HSNRF of fetal origin. The hypothesis can be tested using an odds ratio test, $L_H$:

$$L_H = \frac{\left(\dfrac{p_1}{1-p_1}\right)}{\left(\dfrac{p_2}{1-p_2}\right)}$$

where $p_1$ is the probability of encountering a hot spot in a probe that is candidate for HSNRF of tissues of interest (as presented in Figure 1(B) for the case of fetal HSNRF) and $p_2$ is the probability of encountering a hot spot in a probe that is not a candidate for HSNRF of tissue of interest (as presented in Figure 1(A)). Values where LH > 1 imply an increased representation of such hot spots in HSNRF-candidate probes. Using a cohort of 96 samples, a value of LH = 1.27 (95% CI: 1.08 - 1.50) was obtained illustrating that it is more likely to obtain hot spots of interest in candidate HSNRF probes.

Confirmation of HSNRF sites can occur via quantitation of the fractions of a mixed sample. Since HSNRF sites are associated with a tissue of origin, then quantitation of the fractions of a mixed sample using HSNRF sites and non-HSNRF sites should provide different estimates. Specifically, in the case of using HSNRF on short fragment sites the contribution of the tissue associated with the particular tissue of origin should be greater than when compared to the value obtained when performing the same measurement using other sites.

Figure 2 shows the fetal fraction estimates using SNP information from fragments that originate from HSNRF sites (y-axis) and compares them to fetal fraction estimates when SNP information is obtained from all fragments, irrespective of their origin (x-axis). Each dot represents a sample. As seen in the figure, all data points lie above the x=y line. This indicates that fetal fraction estimates from HSNRF sites result in higher value, indicating an increased presence of fetal material and thus confirming the existence of the HSNRF sites.

**Claims**

1. A method for determining the origin of a nucleic acid fragment, or detecting a nucleic acid fragment, in a mixture of nucleic acid fragments, wherein the mixture comprises maternal and fetal cell-free DNA, or tumor and non-tumor cell-free DNA, the method comprising the steps of

a) providing a mixture of fragmented nucleic acids stemming from a human subject,
b) preparing a sequencing library from the mixture of fragmented nucleic acids,
c) hybridizing one or more probes to at least one location in said library, wherein the mixture of fragmented nucleic acids comprises a genomic region comprising, at a distance of less than 300 bp, genomic bases at which the frequency of being an end-point of a read is significantly different, with a p-value of less than 0.05, between two tissue types present in a mixture of cell-free DNA (cfDNA) and said probe covers said genomic region,
wherein the probes are double-stranded probes and,

   i. each probe is between 100-500 base pairs in length,
   ii. each denatured probe has a 5'-end and a 3'-end,
   iii. each probe binds to a genomic region comprising, at a distance of less than 300 bp, genomic bases differentiating two tissue types present in a mixture of cfDNA at least 10 base pairs away, on both the 5'-end and the 3'-end, from regions harboring copy number variations (CNVs), segmental duplications or repetitive DNA elements, and
   iv. the GC content of each probe is between 10% and 80%,

d) isolating one or more fragmented nucleic acids from the mixture that are bound by the one or more probes,
e) sequencing the enriched library wherein a duplication rate of the sequencing library from the template DNA fragments is more than 5%,
f) determining the fragment size of a fragmented nucleic acid without alignment on any reference genome, by using the sequence similarity of sequenced reads by detecting the amount of overlap and/or homology of paired-reads or the length of single reads,
g) determining the sequence of the, at least 20bp, outermost nucleotides of the fragmented nucleic acid, and
h) utilizing the information from steps (f) and (g) in order to

   i. determine whether the size of the nucleic acid fragment is less than 150 bp, or
   ii. determine whether the size of the nucleic acid fragment is greater than or equal to 150 bp and the sequence derived from step (g) overlaps said genomic region, and

i) categorizing the fragment as of placental or tumor origin if the criteria in step (h)(i) or step (h)(ii) are met or categorizing the fragment as of maternal or non-tumor origin if neither of the criteria in step (h)(i) and step (h)(ii) are met.

2. The method according to claim 1, wherein the nucleic acid fragment is circulating cell-free DNA or RNA.

3. The method according to any of the claims 1 and 2, wherein the nucleic acid fragments are selected from the groups comprising:

   i. embryonic DNA and maternal DNA,
   ii. tumor derived DNA and non-tumor derived DNA,
   iii. pathogen DNA and host DNA,
   iv. DNA derived from a transplanted organ and DNA derived from the host.

4. The method according to any of the preceding claims, wherein the nucleic acid fragment to be detected or the origin of which is to be determined is present in the mixture at a concentration lower than a nucleic acid fragment from the same genetic locus but of different origin.

5. The method according to claim 4, wherein the nucleic acid fragment to be detected or the origin of which is to be determined and the nucleic acid fragment from the same genetic locus but of different origin are present in the mixture at a ratio selected from the group of, 1:2, 1:4, 1:10, 1:20, 1:50, 1:100, 1:200, 1:500, 1:1000, 1:2000 and 1:5000.

6. The method according to any of the preceding claims, wherein the probes are fixed to a support.

7. The method according to any of the preceding claims, wherein the probes are biotinylated and are bound to streptavidin-coated magnetic beads.

8. The method according to any of the preceding claims, wherein the GC content of the probes or probes is between 10% and 70%, preferably 15% and 60%, more preferably 20% and 50%.

9. A method for isolating one or more nucleic acid fragments from a mixture of nucleic acid fragments, comprising the steps of:

   a. providing a mixture of fragmented nucleic acids, preferably DNAs, stemming from a human subject;
   b. hybridizing one or more probes to at least one location in the nucleic acid fragments, where a genomic region comprising, at a distance of less than 300 bp, genomic bases at which the frequency of being an end-point of a read is significantly different, with a p-value of less than 0.05, between two tissue types present in a mixture of cfDNA lies, wherein:

   i. each probe is between 100-500 base pairs in length;
   ii. each denatured probe has a 5'-end and a 3'-end;
   iii. each probe binds to a genomic region comprising, at a distance of less than 300 bp, genomic bases differentiating two tissue types present in a mixture of cfDNA at least 10 base pairs away, on both the 5'-end and the 3'-end, from regions harboring copy number variations (CNVs), segmental duplications or repetitive DNA elements; and
   iv. the GC content of each probe is between 10% and 80%, or

   c. amplifying one or more locations from the nucleic acid fragments, wherein the primers for the amplification lie adjacent to said genomic region.

10. Kit for determining the origin of a nucleic acid fragment in a mixture of nucleic acid fragments for the use in a method according to claims 1 to 9, comprising:

   a. probes that hybridize to at least one location in the nucleic acid fragment, wherein said at least one location partially or completely encompasses a genomic region comprising, at a distance of less than 300 bp, genomic bases at which the frequency of being an end-point of a read is significantly different, with a p-value of less than 0.05, between two tissue types present in a mixture of cfDNA, wherein

   i. each probe is between 100-500 base pairs in length;
   ii. each denatured probe has a 5'-end and a 3'-end;
   iii. each probe binds to a genomic region comprising, at a distance of less than 300 bp, genomic bases differentiating two tissue types present in a mixture of cfDNA at least 10 base pairs away, on both the 5'-end and the 3'-end, from regions harboring copy number variations (CNVs), segmental duplications or repetitive DNA elements; and
   iv. the GC content of each probe is between 10% and 80%; and, optionally,

   b. reagents and/or software for performing the method described according to claims 1 to 9 and a determination and/or detection method.

**Patentansprüche**

1. Verfahren zur Bestimmung der Herkunft eines Nukleinsäurefragments oder zum Nachweisen eines Nukleinsäurefragments in einer Mischung von Nukleinsäurefragmenten, wobei die Mischung mütterliche und fötale zellfreie DNA oder tumorale und nicht-tumorale zellfreie DNA umfasst, wobei das Verfahren die folgenden Schritte umfasst:

a) Bereitstellen einer Mischung fragmentierter Nukleinsäuren, die von einem menschlichen Subjekt stammen,
b) Herstellen einer Sequenzierungsbibliothek aus der Mischung fragmentierter Nukleinsäuren,
c) Hybridisieren einer oder mehrerer Sonden an mindestens einer Stelle in der Bibliothek, wobei die Mischung fragmentierter Nukleinsäuren eine genomische Region umfasst, die in einem Abstand von weniger als 300 bp genomische Basen umfasst, bei denen die Häufigkeit, ein Endpunkt eines Reads zu sein, mit einem p-Wert von weniger als 0,05 zwischen zwei Gewebetypen, die in einer Mischung aus zellfreier DNA (cfDNA) vorhanden sind, und die Sonde die genomische Region abdeckt,
wobei die Sonden doppelsträngige Sonden sind und

i. jede Sonde zwischen 100 und 500 Basenpaare lang ist,
ii. jede denaturierte Sonde ein 5'-Ende und ein 3'-Ende aufweist,
iii. jede Sonde an eine genomische Region bindet, die, in einem Abstand von weniger als 300 bp, Genombasen umfasst, die zwei in einer Mischung aus cfDNA vorhandene Gewebetype unterscheiden, die mindestens 10 Basenpaare entfernt sind, sowohl am 5'-Ende als auch am 3'-Ende, von Abschnitte, die Kopienzahlvariationen (CNVs), segmentale Duplikationen oder repetitive DNA-Elemente enthalten, und
iv. der GC-Gehalt jeder Sonde zwischen 10% und 80% liegt,

d) Isolieren einer oder mehrerer fragmentierter Nukleinsäuren aus der Mischung, die an die eine oder mehrere Sonden gebunden ist,
e) Sequenzieren der angereicherten Bibliothek, wobei die Duplikationsrate der Sequenzierungsbibliothek aus den Template-DNA-Fragmenten mehr als 5% beträgt,
f) Bestimmen der Fragmentgröße einer fragmentierten Nukleinsäure ohne Ausrichtung an einem Referenzgenom unter Verwendung der Sequenzähnlichkeit sequenzierter Reads durch Erfassen des Ausmaßes der Überlappung und/oder Homolgie gepaarter Reads oder der Länge einzelner Reads,
g) Bestimmen der Sequenz der mindestens 20 bp äußeren Nukleotide der fragmentierten Nukleinsäure und
h) Verwenden der Informationen aus den Schritte (f) und (g), um

i. zu bestimmen, ob die Größe des Nukleinsäurefragments weniger als 150 bp beträgt, oder
ii. zu bestimmen, ob die Größe des Nukleinsäurefragments größer oder gleich 150 bp ist und die aus Schritt (g) abgeleitete Sequenz die genomische Region überlappt, und

i) Kategorisieren des Fragments als aus der Plazenta oder einem Tumor stammend, wenn die Kriterien in Schritt (h)(i) oder Schritt (h)(ii) erfüllt sind, oder Kategorisieren des Fragments als aus der Mutter oder nicht aus einem Tumor stammend, wenn keines der Kriterien in Schritt (h)(i) und Schritt (h)(ii) erfüllt ist.

2. Verfahren nach Anspruch 1, wobei das Nukleinsäurefragment zirkulierende zellfreie DNA oder RNA ist.

3. Verfahren nach einem der Ansprüche 1 und 2, wobei die Nukleinsäurefragmente aus den Gruppen ausgewählt sind, umfassend:

i. embryonale DNA und mütterliche DNA,
ii. aus Tumoren stammende DNA und nicht aus Tumoren stammende DNA,
iii. pathogene DNA und Wirts-DNA,
iv. DNA aus einem transplantierten Organ und DNA aus dem Wirt stammend.

4. Verfahren nach einem der vorherigen Ansprüche, wobei das nachzuweisende Nukleinsäurefragment oder dessen Ursprung in der Mischung in einer Konzentration vorliegt, die geringer ist als die eines Nukleinsäurefragments aus demselben genetischen Lokus, jedoch unterschiedlichen Ursprungs.

5. Verfahren nach Anspruch 4, wobei das nachzuweisende Nukleinsäurefragment oder dessen Ursprung zu bestimmen ist und das Nukleinsäurefragment aus demselben genetischen Lokus, jedoch unterschiedlichen Ursprungs, in der Mischung in einem Verhältnis vorhanden sind, das aus der Gruppe ausgewählt ist, die aus 1:2, 1:4, 1:10, 1:20, 1:50, 1:100, 1:200, 1:500, 1:1000, 1:2000 und 1:5000 besteht.

**6.** Verfahren nach einem der vorherigen Ansprüche, wobei die Sonden an einem Träger befestigt sind.

**7.** Verfahren nach einem der vorherigen Ansprüche, wobei die Sonden biotinyliert sind und an Streptavidin-beschichtete Magnetkügelchen gebunden sind.

**8.** Verfahren nach einem der vorherigen Ansprüche, wobei der GC-Gehalt der Sonden oder der Sonde zwischen 10% und 70%, vorzugsweise zwischen 15% und 60%, noch bevorzugter zwischen 20% und 50%, liegt.

**9.** Verfahren zum Isolieren eines oder mehrerer Nukleinsäurefragmente aus einer Mischung von Nukleinsäurefragmenten, umfassend die folgenden Schritte:

a. Bereitstellen einer Mischung fragmentierter Nukleinsäuren, vorzugsweise DNAs, die von einem menschlichen Subjekt stammen;
b. Hybridisieren einer oder mehrerer Sonden an mindestens einer Stelle in den Nukleinsäurefragmenten, wobei eine genomische Region, die in einem Abstand von weniger als 300 bp genomische Basen umfasst, bei denen die Häufigkeit, ein Endpunkt eines Reads zu sein, signifikant unterschiedlich ist, mit einem p-Wert von weniger als 0,05 zwischen zwei Gewebetypen, die in einer Mischung von cfDNA vorhanden sind, wobei:

i. jede Sonde zwischen 100 und 500 Basenpaaren lang ist;
ii. jede denaturierte Sonde ein 5'-Ende und ein 3'-Ende aufweist;
iii. jede Sonde an eine genomische Region bindet, die, in einem Abstand von weniger als 300 bp, genomische Basen umfasst, die zwei in einer cfDNA-Mischung vorhandene Gewebetypen unterscheiden, die mindestens 10 Basenpaare entfernt sind, sowohl am 5'-Ende als auch am 3'-Ende, von Regionen, die Kopienzahlvariationen (CNVs), segmentale Duplikationen oder repetitive DNA-Elemente enthalten; und
iv. der GC-Gehalt jeder Sonde zwischen 10% und 80% liegt, oder

c. Amplifizieren einer oder mehrerer Stellen aus den Nukleinsäurefragmenten, wobei die Primer für die Amplifikation benachbart zu der genomischen Region liegen.

**10.** Kit zur Bestimmung des Ursprungs eines Nukleinsäurefragments in einer Mischung von Nukleinsäurefragmenten zur Verwendung gemäß den Ansprüchen 1 bis 9, umfassend:

a. Sonden, die an mindestens einer Stelle im Nukleinsäurefragment hybridisieren, wobei die mindestens eine Stelle teilweise oder vollständig eine genomische Region umfasst, an denen die Häufigkeit, ein Endpunkt eines Reads zu sein, mit einem p-Wert von weniger als 0,05 signifikant unterschiedlich ist, zwischen zwei Gewebetypen, die in einer Mischung von cfDNA vorhanden sind, wobei

i. jede Sonde zwischen 100 und 500 Basenpaaren lang ist;
ii. jede denaturierte Sonde ein 5'-Ende und ein 3'-Ende aufweist;
iii. jede Sonde an eine genomische Region bindet, die, in einem Abstand von weniger als 300 bp, genomische Basen umfasst, die zwei in einer cfDNA-Mischung vorhandene Gewebetypen unterscheiden, die mindestens 10 Basenpaare entfernt sind, sowohl am 5'-Ende als auch am 3'-Ende, von Regionen, die Kopienzahlvariationen (CNVs), segmentale Duplikationen oder repetitive DNA-Elemente enthalten; und
iv. der GC-Gehalt jeder Sonde zwischen 10% und 80% liegt; und optional

b. Reagenzien und/oder Software zur Durchführung des gemäß den Ansprüchen 1 bis 9 beschriebenen Verfahrens und eines Bestimmungs- und/oder Nachweisverfahrens.

## Revendications

**1.** Procédé d' pour déterminer l'origine d'un fragment d'acide nucléique, ou détecter un fragment d'acide nucléique, dans un mélange de fragments d'acide nucléique , dans lequel le mélange comprend de l'ADN maternel et fœtal sans cellules , ou de l'ADN tumoral et non tumoral sans cellules, le procédé comprenant les étapes consistant à

a) fournir un mélange d'acides nucléiques fragmentés provenant d'un sujet humain ,
b) préparer une bibliothèque de séquençage à partir du mélange d'acides nucléiques fragmentés,
c) hybrider une ou plusieurs sondes à au moins un emplacement dans ladite bibliothèque, dans lequel le mélange d'acides nucléiques fragmentés comprend une région génomique comprenant, à une distance inférieure à 300 pb, des bases génomiques au niveau desquelles la fréquence d'être un point final d'une lecture est significativement différente, avec une valeur p inférieure à 0,05, entre deux types de tissus présents dans un mélange d'ADN acellulaire (cfDNA ) et ladite sonde couvre ladite région génomique ,

dans lequel les sondes sont des sondes double brin et,

i. chaque sonde a une longueur comprise entre 100 et 500 paires de bases,

ii. chaque sonde dénaturée a une extrémité 5' et une extrémité 3',

iii. chaque sonde se lie à une région génomique comprenant, à une distance inférieure à 300 pb, des bases génomiques différenciant deux types de tissus présents dans un mélange d'ADNcf à au moins 10 paires de bases de distance, à la fois à l'extrémité 5' et à l'extrémité 3', des régions abritant des variations du nombre de copies (CNV), des duplications segmentaires ou des éléments d'ADN répétitifs, et

iv. la teneur en GC de chaque sonde est comprise entre 10 % et 80 % d' ,

d) isoler un ou plusieurs acides nucléiques fragmentés du mélange qui sont liés par une ou plusieurs sondes ,

e) séquençant la bibliothèque enrichie dans laquelle un taux de duplication de la bibliothèque de séquençage à partir des fragments d'ADN matrice est supérieur à 5 %,

f) déterminer la taille des fragments d'un acide nucléique fragmenté sans alignement sur aucun génome de référence, en utilisant la similarité de séquence des lectures séquencées en détectant la quantité de chevauchement et/ou d'homologie des lectures appariées ou la longueur des lectures simples,

g) déterminer la séquence des nucléotides les plus externes, d'au moins 20 pb, de l'acide nucléique fragmenté, et

h) utiliser les informations des étapes (f) et (g) afin de

i. déterminer si la taille du fragment d'acide nucléique est inférieure à 150 pb, ou

ii. déterminer si la taille du fragment d'acide nucléique est supérieure ou égale à 150 pb et si la séquence dérivée de l'étape (g) chevauche ladite région génomique , et

i) classer le fragment comme étant d'origine placentaire ou tumorale si les critères de l'étape (h)(i) ou de l'étape (h)(ii) sont remplis ou classer le fragment comme étant d'origine maternelle ou non tumorale si aucun des critères de l'étape (h)(i) et de l'étape (h)(ii) n'est rempli .

2. Procédé selon la revendication 1, dans lequel le fragment d'acide nucléique est de l'ADN ou de l'ARN circulant libre de cellules.

3. Procédé selon l'une quelconque des revendications 1 et 2, dans lequel les fragments d'acide nucléique sont choisis parmi les groupes comprenant :

i. l'ADN embryonnaire et l'ADN maternel,

ii. l'ADN dérivé de tumeurs et l'ADN non dérivé de tumeurs,

iii. l'ADN pathogène et l'ADN hôte,

iv. l'ADN dérivé d'un organe transplanté et l'ADN dérivé de l'hôte.

4. Procédé selon l'une quelconque des revendications précédentes, dans lequel le fragment d'acide nucléique à détecter ou dont l'origine doit être déterminée est présent dans le mélange à une concentration inférieure à celle d'un fragment d'acide nucléique provenant du même locus génétique mais d'origine différente.

5. Procédé selon la revendication 4, dans lequel le fragment d'acide nucléique à détecter ou dont l'origine doit être déterminée et le fragment d'acide nucléique provenant du même locus génétique mais d'origine différente sont présents dans le mélange à un rapport choisi dans le groupe constitué de 1:2, 1:4, 1:10, 1:20, 1:50, 1:100, 1:200, 1:500, 1:1000, 1:2000 et 1:5000.

6. Procédé selon l'une quelconque des revendications précédentes, dans lequel les sondes sont fixées à un support.

7. Procédé selon l'une quelconque des revendications précédentes, dans lequel les sondes sont biotinylées et sont liées à des billes magnétiques recouvertes de streptavidine.

8. Procédé selon l'une quelconque des revendications précédentes, dans lequel la teneur en GC des sondes ou de la sonde est comprise entre 10 % et 70 %, de préférence entre 15 % et 60 %, et plus préférablement entre 20 % et 50 %.

9. Procédé pour isoler un ou plusieurs fragments d'acide nucléique à partir d'un mélange de fragments d'acide nucléique , comprenant les étapes suivantes :

a. fournir un mélange d'acides nucléiques fragmentés, de préférence des ADN, provenant d'un sujet humain ;

b. hybrider une ou plusieurs sondes à au moins un emplacement dans les fragments d'acide nucléique, où une région génomique comprenant, à une distance inférieure à 300 pb, des bases génomiques auxquelles la fréquence d'être un point final d'une lecture est significativement différente, avec une valeur p inférieure à 0,05, entre deux types de tissus présents dans un mélange d'ADN libre circulant se trouve, dans lequel :

    i. chaque sonde a une longueur comprise entre 100 et 500 paires de bases ;

    ii. chaque sonde dénaturée a une extrémité 5' et une extrémité 3' ;

    iii. chaque sonde se lie à une région génomique comprenant, à une distance inférieure à 300 pb, des bases génomiques différenciant deux types de tissus présents dans un mélange d'ADNcf à au moins 10 paires de bases de distance, à la fois à l'extrémité 5' et à l'extrémité 3', des régions abritant des variations du nombre de copies (CNV), des duplications segmentaires ou des éléments d'ADN répétitifs ; et

    iv. la teneur en GC de chaque sonde est comprise entre 10 % et 80 %, ou

c. amplifiant un ou plusieurs emplacements à partir des fragments d'acide nucléique, dans lequel les amorces pour l'amplification sont adjacentes à ladite région génomique.

**10.** Kit pour déterminer l'origine d'un fragment d'acide nucléique dans un mélange de fragments d'acide nucléique destiné à être utilisé dans un procédé selon les revendications 1 à 9, comprenant :

    a. sondes qui s'hybrident à au moins un emplacement dans le fragment d'acide nucléique, , ledit au moins un emplacement englobant partiellement ou complètement une région génomique comprenant, à une distance inférieure à 300 pb , des bases génomiques à dont la fréquence d'être un point final d'une lecture est significativement différente, avec une valeur p inférieure à 0,05, entre deux types de tissus présents dans un mélange d'ADN libre circulant , dans lequel

    i. chaque sonde a une longueur comprise entre 100 et 500 paires de bases ;

    ii. chaque sonde dénaturée a une extrémité 5' et une extrémité 3' ;

    iii. chaque sonde se lie à une région génomique comprenant, à une distance inférieure à 300 pb, des bases génomiques différenciant deux types de tissus présents dans un mélange d'ADN libre circulant à au moins 10 paires de bases, à la fois à l'extrémité 5' et à l'extrémité 3', des régions présentant des variations du nombre de copies (CNV), des duplications segmentaires ou des éléments d'ADN répétitifs ; et

    iv. la teneur en GC de chaque sonde est comprise entre 10 % et 80 % ; et, éventuellement,

b. des réactifs et/ou un logiciel pour mettre en œuvre le procédé décrit selon les revendications 1 à 9 et un procédé de détermination et/ou de détection.

**FIG. 1**

**FIG. 2**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2018081130 A1

- WO 2017181146 A1

**Non-patent literature cited in the description**

- **HEITZER E. et al.** *Nat. Rev. Genet.*, February 2019, vol. 20 (2), 71-88

- **KOUMBARIS et al.** *Clinical chemistry*, 2016, vol. 62 (6), 848-855